# EUROPEAN PATENT APPLICATION

(11) **EP 3 092 939 A1**
(43) Date of publication of application: **16.11.2016**
(21) Application number: 15782493.9
(22) Date of filing: 27.02.2015
(51) Int. Cl.: A61B 1/00, G02B 23/24, G02B 26/10

(54) **OPTICAL SCANNING DEVICE AND SCANNING-TYPE ENDOSCOPE**

(30) Priority: 22.04.2014 JP 2014088464
(71) Applicant: Olympus Corporation, Tokyo 192-8507 (JP)
(72) Inventor: OKITA, Yoshinari, Hachioji-shi, Tokyo 192-8507 (JP)
(74) Representative: Gunzelmann, Rainer
(86) International application number: PCT/JP2015/055841
(87) International publication number: WO 2015/163001

(57) **Abstract**

An optical scanning apparatus includes a fixing member having a through hole at a center portion and configured to fix an exit end portion in a state penetrating through the through hole, a holding member configured to hold the fixing member in a cantilevered manner, and an actuator section including a first drive section, provided on an outer surface of the fixing member, configured to swing, in a first direction, the exit end portion protruding from a distal end portion of the fixing member, and a second drive section, provided on an outer surface of the fixing member, configured to swing, in a second direction different from the first direction, the exit end portion protruding from the distal end portion of the fixing member, where at least one of the fixing member, the holding member, and the actuator section includes a part that is formed in such a way that a shape seen in the first direction from a center axis passing through a center of the through hole and a shape seen in the second direction from the center axis are asymmetric.

## Description

### Technical Field

The present invention relates to an optical scanning apparatus and a scanning endoscope, and more particularly, to an optical scanning apparatus and a scanning endoscope used for acquiring an image by scanning an object.

### Background Art

With respect to endoscopes in a medical field, various technologies for reducing a diameter of an insertion section to be inserted into a body cavity of a subject are proposed so as to reduce a burden on the subject. As an example of such a technology, a scanning endoscope is known which does not include a solid-state image pickup device at a part corresponding to the insertion section mentioned above. Moreover, a scanning fiber device akin to the scanning endoscope mentioned above is disclosed in Japanese Patent Application Laid-Open Publication No. 2010-527028, for example.

More specifically, Japanese Patent Application Laid-Open Publication No. 2010-527028 discloses a scanning fiber device configured to two-dimensionally scan, in a spiral scan pattern, an object irradiated with illumination light emitted from a light source, by holding an exit end portion of an optical fiber for transmitting the illumination light in a cantilevered manner by an actuator tube including a piezoelectric tube and swinging the exit end portion.

Furthermore, with respect to the scanning endoscope as described above, there is known a scanning method of two-dimensionally scanning an object in a Lissajous scan pattern, for example, in addition to the spiral scan pattern disclosed in Japanese Patent Application Laid-Open Publication No. 2010-527028, for example.

However, Japanese Patent Application Laid-Open Publication No. 2010-527028 does not mention any specific method for two-dimensionally scanning an object in the Lissajous scan pattern. Accordingly, in the case of applying the Lissajous scan pattern to the scanning fiber device disclosed in Japanese Patent Application Laid-Open Publication No. 2010-527028, the following problems may occur, for example; the amplitude of an optical fiber does not reach a desired amplitude even when a predetermined voltage is applied to a piezoelectric tube, or excessively high voltage has to be applied to the piezoelectric tube in order to make the amplitude of the optical fiber reach a desired amplitude.

That is, according to the configuration disclosed in Japanese Patent Application Laid-Open Publication No. 2010-527028, the problem described above leads to a problem that reduction in efficiency at the time of scanning an object in the Lissajous scan pattern is inevitable.

The present invention has been made in view of the above circumstance, and has its aim to provide an optical scanning apparatus and a scanning endoscope which are capable of increasing the efficiency at the time of scanning an object in the Lissajous scan pattern.

### Disclosure of Invention

### Means for Solving the Problem

An optical scanning apparatus of an aspect of the present invention includes a fixing member having, at a center portion, a cylindrical through hole allowing penetration of a light guide member that is configured to guide light entering an incident end portion and to emit the light from an exit end portion, the fixing member being configured to fix the exit end portion in a state penetrating through the through hole, a holding member configured to hold the fixing member at a proximal end portion of the fixing member in a cantilevered manner, and an actuator section including a first drive section, provided on an outer surface of the fixing member, configured to swing, in a first direction, the exit end portion protruding from a distal end portion of the fixing member, and a second drive section, provided on an outer surface of the fixing member, configured to swing, in a second direction different from the first direction, the exit end portion protruding from the distal end portion of the fixing member, where at least one of the fixing member, the holding member, and the actuator section includes a part that is formed in such a way that a shape seen in the first direction from a center axis passing through a center of the through hole and a shape seen in the second direction from the center axis are asymmetric.

A scanning endoscope of an aspect of the present invention includes an insertion section formed to have a shape allowing insertion into a body cavity, a light guide member, inserted through the insertion section, configured to guide light entering an incident end portion and to emit the light from an exit end portion, a fixing member having, at a center portion, a cylindrical through hole allowing penetration of the light guide member, the fixing member being configured to fix the exit end portion in a state penetrating through the through hole, a holding member configured to hold the fixing member at a proximal end portion of the fixing member in a cantilevered manner, and an actuator section including a first drive section, provided on an outer surface of the fixing member, configured to swing, in a first direction, the exit end portion protruding from a distal end portion of the fixing member, and a second drive section, provided on an outer surface of the fixing member, configured to swing, in a second direction different from the first direction, the exit end portion protruding from the distal end portion of the fixing member, where at least one of the fixing member, the holding member, and the actuator section includes a part that is formed in such a way that a shape seen in the first direction from a center axis passing through a center of the through hole and a shape seen in the second direction from the center axis are asymmetric.

### Brief Description of the Drawings

Fig. 1 is a diagram showing a configuration of main sections of a scanning endoscope system including a scanning endoscope according to an embodiment of the present invention;
Fig. 2A is a diagram showing an example configuration of an optical scanning apparatus according to a first embodiment;
Fig. 2B is a diagram showing an example configuration seen from a direction indicated by an arrow AR1 in Fig. 2A;
Fig. 3 is a diagram showing examples of signal waveforms of drive signals supplied to an actuator section of a scanning endoscope according to the embodiment of the present invention;
Fig. 4 is a diagram showing an example of a Lissajous scan pattern;
Fig. 5 is a diagram showing examples of vibration efficiency in an X-axis direction and a Y-axis direction at the time of scanning an object in a Lissajous pattern by using the scanning endoscope according to the embodiment of the present invention;
Fig. 6A is a diagram showing an example configuration of an optical scanning apparatus according to an example modification of the first embodiment;
Fig. 6B is a diagram showing an example configuration seen from a direction indicated by an arrow AR2 in Fig. 6A;
Fig. 7A is a diagram showing an example configuration of an optical scanning apparatus according to an example modification of the first embodiment, different from that shown in Figs. 6;
Fig. 7B is a diagram showing an example configuration seen from a direction indicated by arrow AR3 in Fig. 7A;
Fig. 8A is a diagram showing an example configuration of an optical scanning apparatus according to an example modification of the first embodiment, different from those shown in Figs. 6 and 7;
Fig. 8B is a diagram showing an example configuration seen from a direction indicated by an arrow AR4 in Fig. 8A;
Fig. 9A is a diagram showing an example configuration of an optical scanning apparatus according to a second embodiment;
Fig. 9B is a diagram showing an example configuration seen from a direction indicated by an arrow AR5 in Fig. 9A;
Fig. 10A is a diagram showing an example configuration of an optical scanning apparatus according to a third embodiment;
Fig. 10B is a diagram showing an example configuration seen from a direction indicated by an arrow AR6 in Fig. 10A;
Fig. 11 A is a diagram showing an example configuration of an optical scanning apparatus according to an example modification of the third embodiment;
Fig. 11B is a diagram showing an example configuration seen from a direction indicated by an arrow AR7 in Fig. 11 A;
Fig. 12A is a diagram showing an example configuration of an optical scanning apparatus according to an example modification of the third embodiment, different from that shown in Fig. 11 A;
Fig. 12B is a diagram showing an example configuration seen from a direction indicated by an arrow AR8 in Fig. 12A;
Fig. 13A is a diagram showing an example configuration of an optical scanning apparatus according to an example modification of the third embodiment, different from those shown in Figs. 11A and 12A;
Fig. 13B is a diagram showing an example configuration seen from a direction indicated by an arrow AR9 in Fig. 13A;
Fig. 14A is a diagram showing an example configuration of an optical scanning apparatus according to an example modification of the third embodiment, different from those shown in Figs. 11A, 12A, and 13A;
Fig. 14B is a diagram showing an example configuration seen from a direction indicated by an arrow AR10 in Fig. 14A;
Fig. 15A is a diagram showing an example configuration of an optical scanning apparatus according to an example modification of the third embodiment, different from those shown in Figs. 11A, 12A, 13A, and 14A; and
Fig. 15B is a diagram showing an example configuration seen from a direction indicated by an arrow AR11 in Fig. 15A.

### Best Mode for Carrying Out the Invention

Hereinafter, embodiments of the present invention will be described with reference to the drawings.

### (First Embodiment)

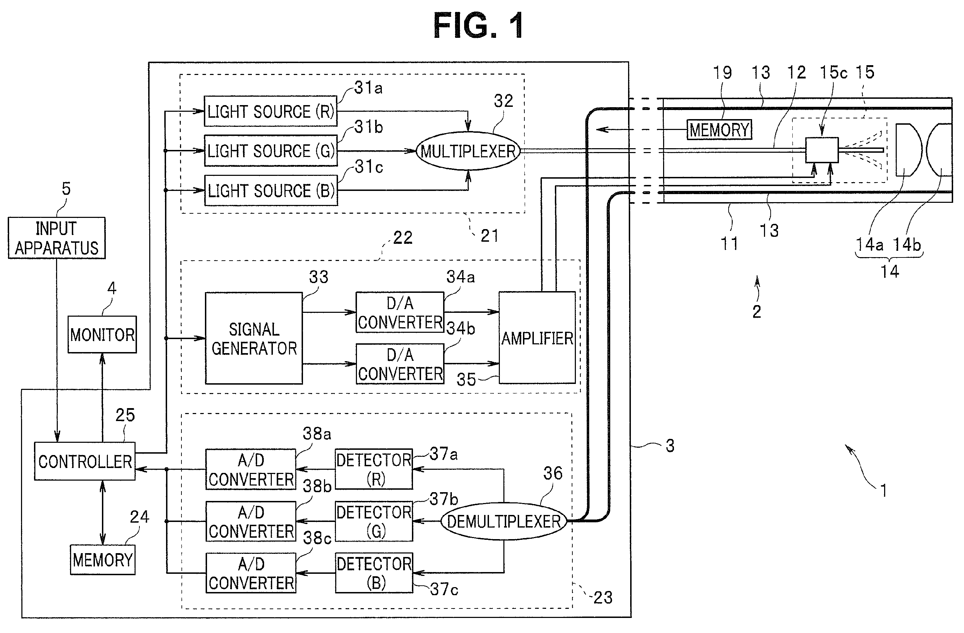

Figs. 1 to 8B are related to a first embodiment of the present invention. Fig. 1 is a diagram showing a configuration of main sections of a scanning endoscope system including a scanning endoscope according to the embodiment of the present invention.

As shown in Fig. 1, for example, a scanning endoscope system 1 is configured from a scanning endoscope 2 which can be inserted into a body cavity of a subject, a main body apparatus 3 which can be connected to the scanning endoscope 2, a monitor 4 to be connected to the main body apparatus 3, and an input apparatus 5 allowing input of information and issuance of instructions to the main body apparatus 3. Note that the input apparatus 5 is not limited to be configured as a separate apparatus from the main body apparatus 3, as shown in Fig. 1, and may alternatively be configured as an interface integrated with the main body apparatus 3, for example.

The scanning endoscope 2 includes an elongated and flexible insertion section 11 which can be inserted into the body cavity of a subject. Note that a connector or the like, not shown, configured to connect the scanning endoscope 2 to the main body apparatus 3 in a freely detachable manner is provided at a proximal end portion of the insertion section 11.

An illumination fiber 12 which functions as a light guide member configured to guide illumination light supplied from a light source unit 21 of the main body apparatus 3 to a collection optical system 14, and a light receiving fiber 13 configured to receive returned light from an object and to guide the light to a detection unit 23 of the main body apparatus 3 are each inserted through a part, inside the insertion section 11, from the proximal end portion to a distal end portion.

An incident end portion, including a light incident surface, of the illumination fiber 12 is disposed at a multiplexer 32 provided inside the main body apparatus 3. Also, an exit end portion, including a light exit surface, of the illumination fiber 12 is included in an optical scanning apparatus 15 described later, and is disposed near a light incident surface of a lens 14a provided at the distal end portion of the insertion section 11 in a manner not fixed by a fixing member or the like.

An incident end portion, including a light incident surface, of the light receiving fiber 13 is disposed fixed to the periphery of a light exit surface of a lens 14b at a distal end surface of the distal end portion of the insertion section 11. Also, an exit end portion, including a light exit surface, of the light receiving fiber 13 is disposed at a demultiplexer 36 provided inside the main body apparatus 3.

The collection optical system 14 includes the lens 14a where illumination light which has passed through the light exit surface of illumination fiber 12 is to enter, and the lens 14b configured to emit the illumination light which has passed through the lens 14a to an object.

The optical scanning apparatus 15 including an actuator section 18 which is driven based on a drive signal outputted from a driver unit 22 of the main body apparatus 3 is provided at the distal end portion of the insertion section 11.

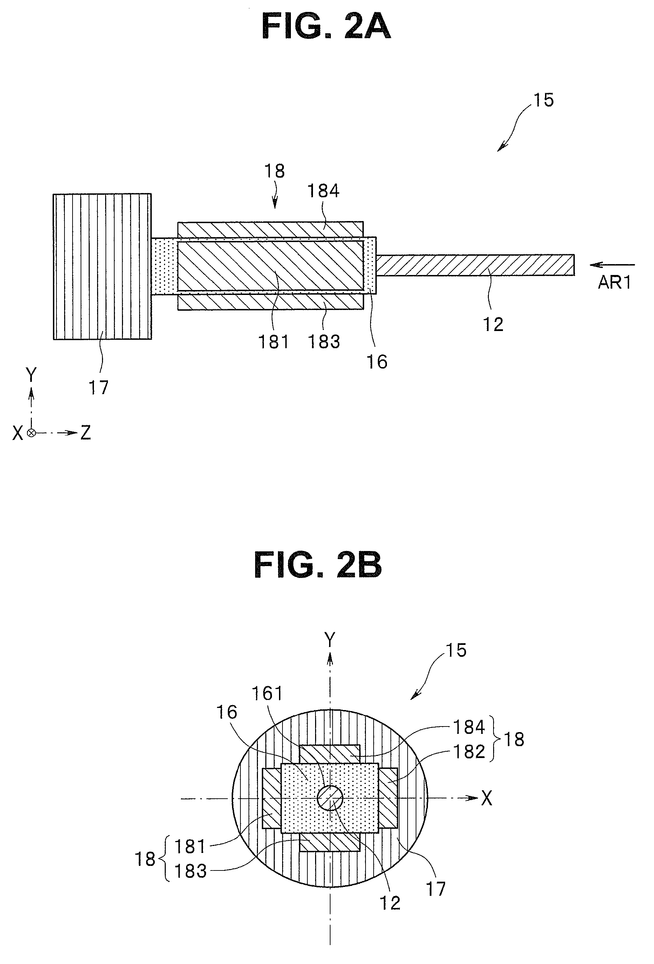

As shown in Figs. 2A and 2B, the optical scanning apparatus 15 includes a fixing member 16 formed as a square column, a holding member 17 configured to hold the fixing member 16 in a cantilevered manner at a proximal end portion of the fixing member 16, and the actuator section 18 provided on outer side surfaces of the fixing member 16. Fig. 2A is a diagram showing an example configuration of an optical scanning apparatus according to the first embodiment. Fig. 2B is a diagram showing an example configuration seen from a direction indicated by an arrow AR1 in Fig. 2A.

Note that, in the following, description will be given assuming that a longitudinal direction of the insertion section 11, a longitudinal direction of the illumination fiber 12, and a longitudinal direction of the fixing member 16 are each parallel to a Z-axis direction shown in Fig. 2A and the like. Also, in the following, description will be given assuming that an X-axis direction shown in Fig. 2A and the like is orthogonal to the Z-axis direction, and that a Y-axis shown in Fig. 2A and the like is orthogonal to the X-axis direction and the Z-axis direction.

The fixing member 16 is formed of ceramics containing zirconia, or metal such as nickel, for example. Furthermore, as shown in Fig. 2B, a circular cylindrical through hole 161 is provided at a center portion of the fixing member 16, along the longitudinal direction, the through hole 161 formed to have an inner diameter approximately the same as an outer diameter of the illumination fiber 12. Note that the fixing member 16 may be formed to have a shape other than a square column, such as a circular column or a polygonal column.

According to the configuration as described above, the exit end portion of the illumination fiber 12 may be fixed in a state as shown in Figs. 2A and 2B, that is, in a state where the exit end portion penetrates the through hole 161 of the fixing member 16 and protrudes from a distal end surface of a distal end portion of the fixing member 16 by a predetermined length. Also, according to the configuration as described above, a center axis passing through the through hole 161 of the fixing member 16 may be treated as a center axis of the optical scanning apparatus 15. Moreover, according to the configuration as described above, a direction of the center axis passing through the center of the through hole 161 of the fixing member 16 and the Z-direction may be treated as the same direction.

Furthermore, as shown in Fig. 2B, the fixing member 16 is formed to have a rectangular shape whose length in the X-axis direction and length in the Y-axis direction are different from each other when the optical scanning apparatus 15 is seen from a direction indicated by the arrow AR1, that is, when the optical scanning apparatus 15 is seen from the side of the light exit surface of the illumination fiber 12.

The holding member 17 is formed of metal such as stainless steel. Also, the holding member 17 functions as a fixing end of the optical scanning apparatus 15, and is configured to hold the optical scanning apparatus 15 at a predetermined position inside the distal end portion of the insertion section 11.

The actuator section 18 is configured by including at least one piezoelectric element configured to swing, in the X-axis direction, the exit end portion of the illumination fiber 12 protruding from the distal end portion of the fixing member 16, and at least one piezoelectric element configured to swing, in the Y-axis direction, the exit end portion of the illumination fiber 12 protruding from the distal end portion of the fixing member 16. More specifically, as shown in Figs. 2A and 2B, for example, the actuator section 18 includes piezoelectric elements 181 and 182 provided in the X-axis direction, and piezoelectric elements 183 and 184 provided in the Y-axis direction.

The piezoelectric elements 181 and 182 are provided at positions, on outer side surfaces of the fixing member 16, facing each other across the illumination fiber 12. Also, the piezoelectric elements 181 and 182 are capable of swinging, in the X-axis direction, the exit end portion of the illumination fiber 12 protruding from the distal end portion of the fixing member 16, by vibrating (repeatedly expanding and contracting while maintaining opposite expansion/contraction states) according to a first drive signal supplied by the driver unit 22 of the main body apparatus 3.

The piezoelectric elements 183 and 184 are provided at positions, on outer side surfaces of the fixing member 16, facing each other across the illumination fiber 12. Also, the piezoelectric elements 183 and 184 are capable of swinging, in the Y-axis direction, the exit end portion of the illumination fiber 12 protruding from the distal end portion of the fixing member 16, by vibrating (repeatedly expanding and contracting while maintaining opposite expansion/contraction states) according to a second drive signal supplied by the driver unit 22 of the main body apparatus 3.

The piezoelectric elements 181 to 184 are formed as cuboids having same length L, width W, and thickness T as one another. Also, the piezoelectric elements 181 to 184 are formed using a same piezoelectric material as one another.

A memory 19 is provided inside the insertion section 11, the memory 19 storing in advance endoscope information including various pieces of information such as individual identification information of the scanning endoscope 2. Note that, according to the present embodiment, a connector (not shown) configured to connect the scanning endoscope 2 to the main body apparatus 3 in a freely detachable manner is desirably provided with the memory 19. Moreover, the endoscope information stored in the memory 19 is read by a controller 25 of the main body apparatus 3 when the scanning endoscope 2 and the main body apparatus 3 are connected together.

Meanwhile, the main body apparatus 3 includes a light source unit 21, a driver unit 22, a detection unit 23, a memory 24, and a controller 25.

The light source unit 21 includes a light source 31 a, a light source 31b, a light source 31 c, and the multiplexer 32.

The light source 31a includes a laser light source, for example, and is configured to emit light in a red wavelength band (hereinafter referred to also as R light) to the multiplexer 32 when the light source 31a is caused to emit light under control of the controller 25.

The light source 31b includes a laser light source, for example, and is configured to emit light in a green wavelength band (hereinafter referred to also as G light) to the multiplexer 32 when the light source 31b is caused to emit light under control of the controller 25.

The light source 31c includes a laser light source, for example, and is configured to emit light in a blue wavelength band (hereinafter referred to also as B light) to the multiplexer 32 when the light source 31c is caused to emit light under control of the controller 25.

The multiplexer 32 is configured to be capable of multiplexing the R light emitted from the light source 31a, the G light emitted from the light source 31b, and the B light emitted from the light source 31c, and of supplying the multiplexed light to the light incident surface of the illumination fiber 12.

The driver unit 22 includes a signal generator 33, D/A converters 34a and 34b, and an amplifier 35.

The signal generator 33 is configured to generate a first drive signal for swinging the exit end portion of the illumination fiber 12 in the X-axis direction under the control of the controller 25, and to output the signal to the D/A converter 34a. The signal generator 33 is configured to also generate a second drive signal for swinging the exit end portion of the illumination fiber 12 in the Y-axis direction under the control of the controller 25, and to output the signal to the D/A converter 34b.

The D/A converter 34a is configured to convert the digital first drive signal outputted from the signal generator 33 into an analog first drive signal, and to output the analog first drive signal to the amplifier 35.

The D/A converter 34b is configured to convert the digital second drive signal outputted from the signal generator 33 into an analog second drive signal, and to output the analog second drive signal to the amplifier 35.

The amplifier 35 is configured to amplify the first and the second drive signals outputted from the D/A converters 34a and 34b, and to output the amplified signals to the actuator section 18.

The detection unit 23 includes a demultiplexer 36, detectors 37a, 37b and 37c, and A/D converters 38a, 38b and 38c.

The demultiplexer 36 is provided with a dichroic mirror or the like, and is configured to separate light emitted from the light exit surface of the light receiving fiber 13 into light of each of color components of R (red), G (green), and B (blue), and to emit the separated light to the detectors 37a, 37b and 37c.

The detector 37a is configured to detect the intensity of R light outputted from the demultiplexer 36, to generate an analog R signal according to the detected intensity of the R light, and to output the analog R signal to the A/D converter 38a.

The detector 37b is configured to detect the intensity of G light outputted from the demultiplexer 36, to generate an analog G signal according to the detected intensity of the G light, and to output the analog G signal to the A/D converter 38b.

The detector 37c is configured to detect the intensity of B light outputted from the demultiplexer 36, to generate an analog B signal according to the detected intensity of the B light, and to output the analog B signal to the A/D converter 38c.

The A/D converter 38a is configured to convert the analog R signal outputted from the detector 37a into a digital R signal, and to output the digital R signal to the controller 25.

The A/D converter 38b is configured to convert the analog G signal outputted from the detector 37b into a digital G signal, and to output the digital G signal to the controller 25.

The A/D converter 38c is configured to convert the analog B signal outputted from the detector 37c into a digital B signal, and to output the digital B signal to the controller 25.

Control programs for controlling the main body apparatus 3, and the like are stored in the memory 24. Also, endoscope information read by the controller 25 of the main body apparatus 3 is stored in the memory 24.

The controller 25 is provided with a CPU and the like, and is configured to operate according to operation of the input apparatus 5, for example.

The controller 25 is configured to read control programs stored in the memory 24, and to control the light source unit 21 and the driver unit 22 based on the control programs which have been read. That is, the actuator section 18 vibrates according to a drive signal supplied from the driver unit 22 under the control of the controller 25 to thereby swing the exit end portion of the illumination fiber 12 in such a way that a position irradiated with illumination light emitted to an object draws a trajectory according to a predetermined scan pattern.

The controller 25 operates to read endoscope information from the memory 19 and to store the information in the memory 24 when the insertion section 11 is electrically connected to the main body apparatus 3.

The controller 25 is configured to generate an image based on the R signal, the G signal, and the B signal outputted from the detection unit 23, and to display the generated image on the monitor 4.

Next, an action of the present embodiment will be described.

First, after connecting each section of the scanning endoscope system 1 and turning on the power, a user, such as a surgeon, issues an instruction to start scanning of an object by operating a predetermined switch of the input apparatus 5.

When the power of the main body apparatus 3 is turned on and the insertion section 11 is electrically connected, the controller 25 reads endoscope information from the memory 19, and stores the information in the memory 24.

For example, when pressing down of a predetermined switch, such as a scanning start switch, provided to the input apparatus 5 is detected, the controller 25 controls the light source unit 21 to supply mixed light (white light) of the R light, the G light, and the B light to the illumination fiber 12 as the illumination light.

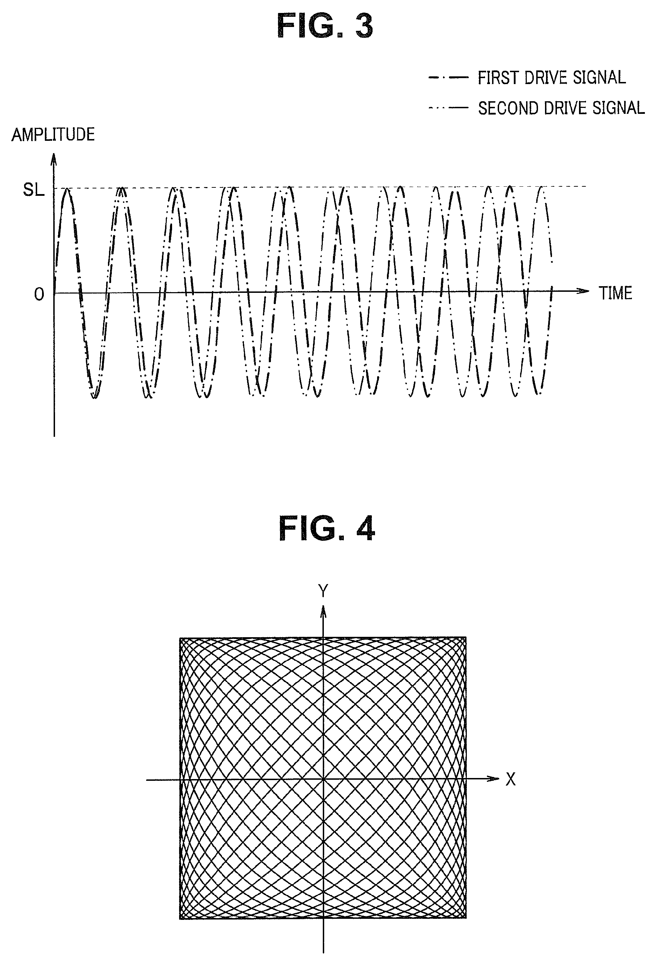

When pressing down of a predetermined switch of the input apparatus 5 is detected, the controller 25 controls the signal generator 33 to generate a first drive signal whose signal waveform is a sine wave with frequency f1 and a predetermined amplitude SL, as shown by a one-dot chain line in Fig. 3, for example. Also, when pressing down of the predetermined switch of the input apparatus 5 is detected, the controller 25 controls the signal generator 33 to generate a second drive signal whose signal waveform is a sine wave with frequency f2, different from the frequency f1, and the amplitude SL, as shown by a broken line in Fig. 3, for example. Fig. 3 is a diagram showing examples of the signal waveforms of drive signals supplied to the actuator section of the scanning endoscope according to the embodiment of the present invention.

Furthermore, when the first drive signal with the signal waveform as shown by the one-dot chain line in Fig. 3 is supplied to the piezoelectric elements 181 and 182 of the actuator section 18, and the second drive signal with the signal waveform as shown by the two-dot chain line in Fig. 3 is supplied to the piezoelectric elements 183 and 184 of the actuator section 18, the exit end portion of the illumination fiber 12 protruding from the distal end portion of the fixing member 16 is swung in a Lissajous pattern, and the surface of the object is scanned in the Lissajous pattern as shown in Fig. 4 according to the swinging. Fig. 4 is a diagram showing an example of the Lissajous scan pattern.

According to the present embodiment, when the fixing member 16 is seen from the side of the light exit surface of the illumination fiber 12, the lengths of the fixing member 16 in the X-axis direction and the Y-axis direction are different from each other. In other words, the fixing member 16 of the present embodiment is formed in such a way that the shape seen in the X-axis direction from the center axis passing though the center of the through hole 161 and the shape seen in the Y-axis direction from the center axis are asymmetric. Therefore, according to the present embodiment, the frequency f1 of the first drive signal may be made to coincide with mechanical resonance frequency frx, in the X-axis direction, of the vibration of the piezoelectric elements 181 and 182, and also, the frequency f2 of the second drive signal may be made to coincide with mechanical resonance frequency fry, in the Y-axis direction, of the vibration of the piezoelectric elements 183 and 184.

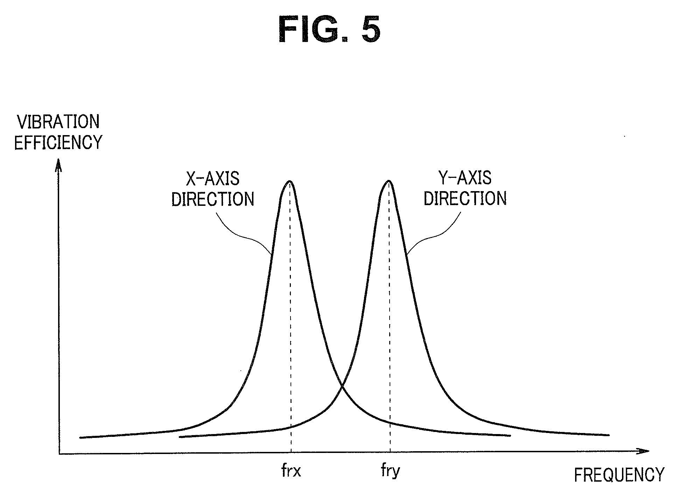

As described above, according to the present embodiment, at the time of scanning an object in the Lissajous pattern, control is performed so as to supply the first drive signal whose frequency f1 is set to frx, for example, to the piezoelectric elements 181 and 182, and therefore, the vibration efficiency in the X-axis direction corresponding to the size of amplitude of the exit end portion of the illumination fiber 12 observed at the time of application of a specific voltage to the piezoelectric elements 181 and 182 may be maximized (see Fig. 5). Also, as described above, according to the present embodiment, at the time of scanning an object in the Lissajous pattern, control is performed so as to supply the second drive signal whose frequency f2 is set to fry, for example, to the piezoelectric elements 183 and 184, and therefore, the vibration efficiency in the Y-axis direction corresponding to the size of amplitude of the illumination fiber 12 observed at the time of application of the specific voltage to the piezoelectric elements 183 and 184 may be maximized (see Fig. 5). Fig. 5 is a diagram showing examples of vibration efficiency in the X-axis direction and the Y-axis direction at the time of scanning an object in the Lissajous pattern by using the scanning endoscope according to the embodiment of the present invention.

Therefore, according to the present embodiment, the sizes of the amplitudes of the drive signals at the time of swinging the illumination fiber 12 at desired amplitudes and in the Lissajous pattern may be optimized, and as a result, the efficiency at the time of scanning an object in the Lissajous scan pattern may be increased.

Next, a configuration according to an example modification of the optical scanning apparatus 15 of the present embodiment will be described. Note that, in the following, for the sake of simplicity, sections different from those of the configuration described above will be mainly described while omitting as appropriate description regarding sections to which the same configuration as the configuration described above can be applied.

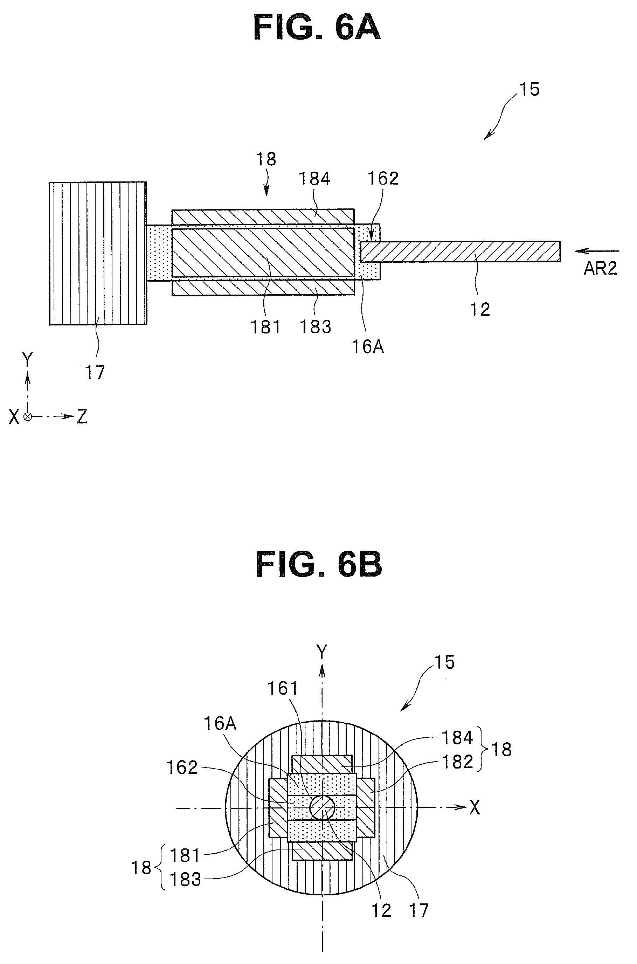

According to a first example modification of the present embodiment, the optical scanning apparatus 15 may be configured by including a fixing member 16A having a shape as shown in Figs. 6A and 6B, instead of the fixing member 16. Fig. 6A is a diagram showing an example configuration of the optical scanning apparatus according to the example modification of the first embodiment. Fig. 6B is a diagram showing an example configuration seen from a direction indicated by an arrow AR2 in Fig. 6A.

For example, as shown in Fig. 6B, the fixing member 16A is formed to have a rectangular shape whose length in the X-axis direction and length in the Y-axis direction are the same when the optical scanning apparatus 15 is seen from the direction indicated by the arrow AR2, that is, when the optical scanning apparatus 15 is seen from the side of the light exit surface of the illumination fiber 12.

Furthermore, for example, as shown in Figs. 6A and 6B, the fixing member 16A has a concave groove section 162 provided on a distal end surface, the groove section 162 having a width approximately the same as the outer diameter of the through hole 161 and formed along the X-axis direction. Note that the shape of the groove section 162 may be other than the concave shape, such as a V-shape or a U-shape. Also, it is enough if the groove section 162 is formed along one of the X-axis direction and the Y-axis direction.

The fixing member 16A of the present example modification is formed in such a way that the shape seen in the X-axis direction from the center axis passing though the center of the through hole 161 and the shape seen in the Y-axis direction from the center axis are asymmetric. Therefore, also in the case of configuring the optical scanning apparatus 15 by using the fixing member 16A of the present example modification, the frequency f1 of the first drive signal may be made to coincide with the mechanical resonance frequency frx, in the X-axis direction, of the vibration of the piezoelectric elements 181 and 182, and also, the frequency f2 of the second drive signal may be made to coincide with the mechanical resonance frequency fry, in the Y-axis direction, of the vibration of the piezoelectric elements 183 and 184. As a result, the efficiency at the time of scanning an object in the Lissajous scan pattern may be increased also in the case of configuring the optical scanning apparatus 15 by using the fixing member 16A of the present example modification.

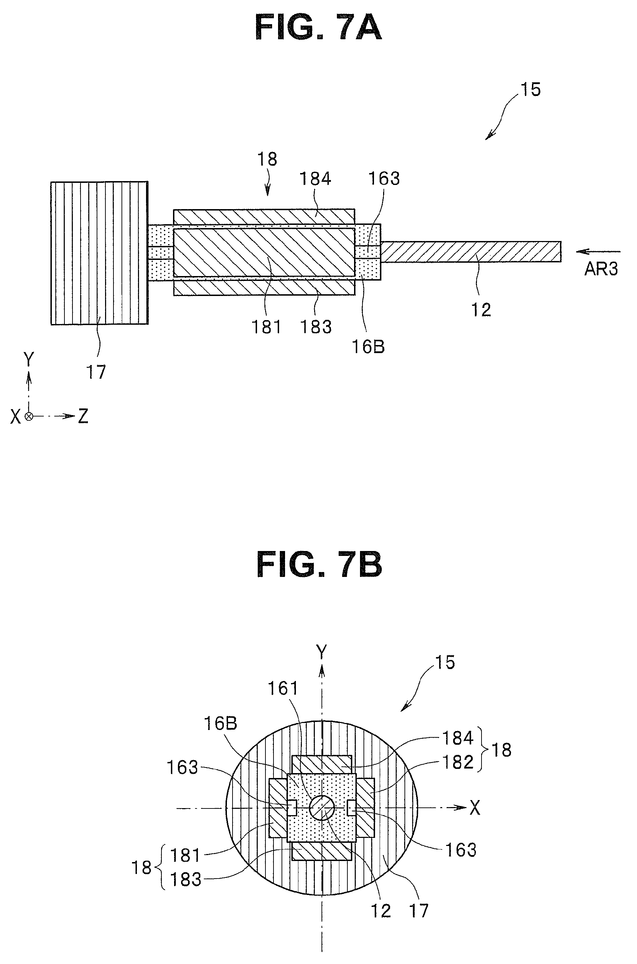

According to a second example modification of the present embodiment, the optical scanning apparatus 15 may be configured by including a fixing member 16B having a shape as shown in Figs. 7A and 7B, instead of the fixing member 16. Fig. 7A is a diagram showing an example configuration of the optical scanning apparatus according to the example modification of the first embodiment, different from that shown in Figs. 6. Fig. 7B is a diagram showing an example configuration seen from a direction indicated by an arrow AR3 in Fig. 7A.

For example, as shown in Fig. 7B, the fixing member 16B is formed to have a rectangular shape whose length in the X-axis direction and length in the Y-axis direction are the same when the optical scanning apparatus 15 is seen from the direction indicated by the arrow AR3, that is, when the optical scanning apparatus 15 is seen from the side of the light exit surface of the illumination fiber 12.

Furthermore, for example, as shown in Figs. 7A and 7B, according to the fixing member 16B, concave groove sections 163 that are formed along the Z-axis direction (the longitudinal direction of the fixing member 16B) are provided one each on at least a part of a side surface where the piezoelectric element 181 is provided and at least a part of a side surface where the piezoelectric element 182 is provided.

Note that the shape of the groove sections 163 may be other than the concave shape, such as a V-shape or a U-shape, as long as the groove sections 163 are formed along the Z-axis direction (the longitudinal direction of the fixing member 16B). Also, it is enough if the groove section 163 is formed on at least one side surface among the side surfaces of the fixing member 16B.

The fixing member 16B of the present example modification is formed in such a way that the shape seen in the X-axis direction from the center axis passing though the center of the through hole 161 and the shape seen in the Y-axis direction from the center axis are asymmetric. Therefore, also in the case of configuring the optical scanning apparatus 15 by using the fixing member 16B of the present example modification, the frequency f1 of the first drive signal may be made to coincide with the mechanical resonance frequency frx, in the X-axis direction, of the vibration of the piezoelectric elements 181 and 182, and also, the frequency f2 of the second drive signal may be made to coincide with the mechanical resonance frequency fry, in the Y-axis direction, of the vibration of the piezoelectric elements 183 and 184. As a result, the efficiency at the time of scanning an object in the Lissajous scan pattern may be increased also in the case of configuring the optical scanning apparatus 15 by using the fixing member 16B of the present example modification.

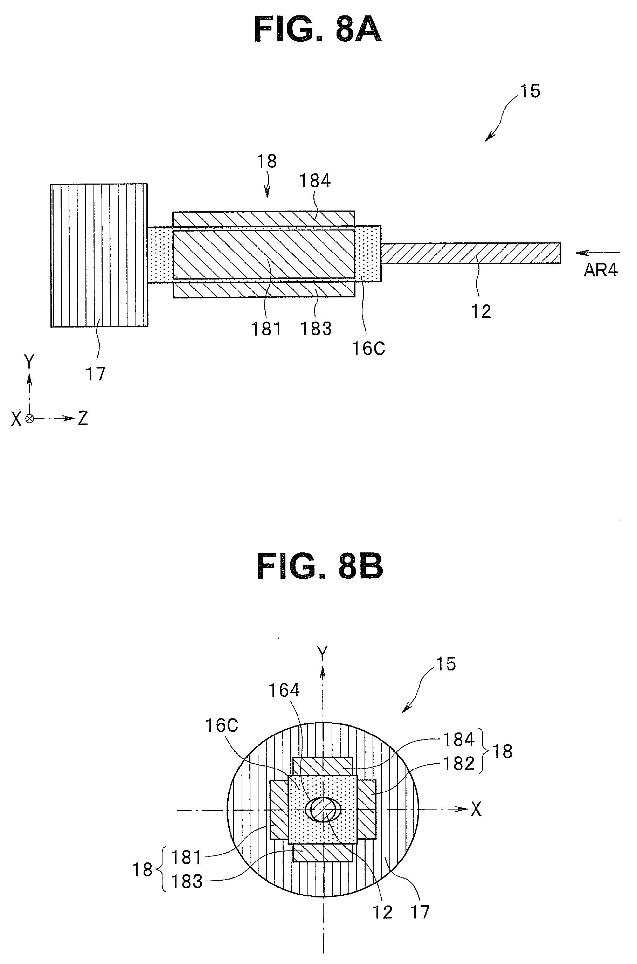

According to a third example modification of the present embodiment, the optical scanning apparatus 15 may be configured by including a fixing member 16C having a shape as shown in Figs. 8A and 8B, instead of the fixing member 16. Fig. 8A is a diagram showing an example configuration of the optical scanning apparatus according to the example modification of the first embodiment, different from those shown in Figs. 6 and 7. Fig. 8B is a diagram showing an example configuration seen from a direction indicated by an arrow AR4 in Fig. 8A.

For example, as shown in Fig. 8B, the fixing member 16C is formed to have a rectangular shape whose length in the X-axis direction and length in the Y-axis direction are the same when the optical scanning apparatus 15 is seen from the direction indicated by the arrow AR4, that is, when the optical scanning apparatus 15 is seen from the side of the light exit surface of the illumination fiber 12.

Furthermore, for example, as shown in Fig. 8B, a through hole 164 having a different shape from the through hole 161 and whose center axis coincides with the center axis of the through hole 161 is provided at a center portion of the fixing member 16C, along the longitudinal direction. More specifically, the through hole 164 has an oval cylindrical shape whose inner diameter in the short axis direction is approximately the same as the outer diameter of the illumination fiber 12. Note that, in the present example modification, clearances may be provided between the inner circumferential surfaces in the long axis direction of the through hole 164 and the outer circumferential surface of the illumination fiber 12, or the inner circumferential surfaces in the long axis direction of the through hole 164 and the outer circumferential surface of the illumination fiber 12 may be bonded together by an adhesive or the like.

The fixing member 16C of the present example modification is formed in such a way that the shape seen in the X-axis direction from the center axis passing though the center of the through hole 164 and the shape seen in the Y-axis direction from the center axis are asymmetric. Therefore, also in the case of configuring the optical scanning apparatus 15 by using the fixing member 16C of the present example modification, the frequency f1 of the first drive signal may be made to coincide with the mechanical resonance frequency frx, in the X-axis direction, of the vibration of the piezoelectric elements 181 and 182, and also, the frequency f2 of the second drive signal may be made to coincide with the mechanical resonance frequency fry, in the Y-axis direction, of the vibration of the piezoelectric elements 183 and 184. As a result, the efficiency at the time of scanning an object in the Lissajous scan pattern may be increased also in the case of configuring the optical scanning apparatus 15 by using the fixing member 16C of the present example modification.

### (Second Embodiment)

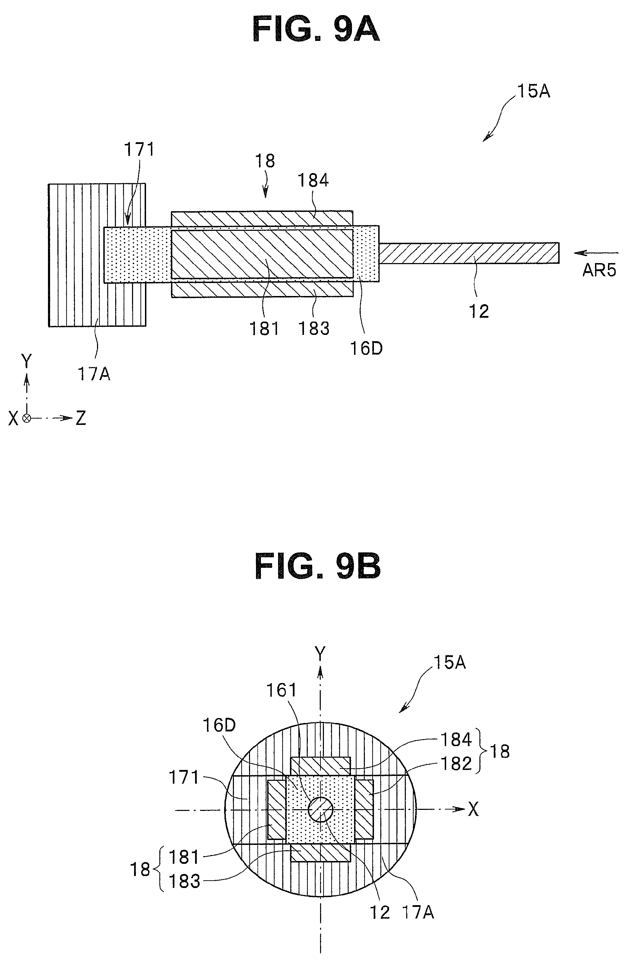

Figs. 9A and 9B are related to a second embodiment of the present invention.

Note that, in the present embodiment, sections having a configuration different from that of the first embodiment will be mainly described while omitting as appropriate detailed description regarding sections having the same configuration as in the first embodiment.

The scanning endoscope 2 of the present embodiment is configured by including an optical scanning apparatus 15A as shown in Figs. 9A and 9B at the distal end portion of the insertion section 11, instead of the optical scanning apparatus 15.

More specifically, as shown in Figs. 9A and 9B, the optical scanning apparatus 15A includes a fixing member 16D formed as a square column, a holding member 17A configured to hold the fixing member 16D in a cantilevered manner at a proximal end portion of the fixing member 16D, and the actuator section 18 provided on outer side surfaces of the fixing member 16D. Fig. 9A is a diagram showing an example configuration of the optical scanning apparatus according to the second embodiment. Fig. 9B is a diagram showing an example configuration seen from a direction indicated by an arrow AR5 in Fig. 9A.

The fixing member 16D is formed of ceramics containing zirconia, or metal such as nickel, for example. Furthermore, as shown in Fig. 9B, the circular cylindrical through hole 161 is provided at a center portion of the fixing member 16D, along the longitudinal direction, the through hole 161 formed to have an inner diameter approximately the same as an outer diameter of the illumination fiber 12. That is, a center axis of the optical scanning apparatus 15A coincides with a center axis of the fixing member 16D in the longitudinal direction and a center axis of the through hole 161. Note that the fixing member 16D may be formed to have a shape other than a square column, such as a circular column or a polygonal column.

According to the configuration of the fixing member 16D described above, the exit end portion of the illumination fiber 12 may be fixed in a state as shown in Figs. 9A and 9B, that is, in a state where the exit end portion penetrates the through hole 161 of the fixing member 16D and protrudes from a distal end surface of a distal end portion of the fixing member 16D by a predetermined length. Also, according to the configuration as described above, the center axis passing through the center of the through hole 161 of the fixing member 16D may be treated as a center axis of the optical scanning apparatus 15A. Moreover, according to the configuration as described above, a direction of the center axis passing through the center of the through hole 161 of the fixing member 16D and the Z-direction may be treated as the same direction.

Furthermore, as shown in Fig. 9B, the fixing member 16D is formed to have a rectangular shape whose length in the X-axis direction and length in the Y-axis direction are the same when the optical scanning apparatus 15A is seen from a direction indicated by the arrow AR5, that is, when the optical scanning apparatus 15A is seen from the side of the light exit surface of the illumination fiber 12.

The holding member 17A is formed of metal such as stainless steel. Also, the holding member 17A functions as a fixing end of the optical scanning apparatus 15A, and is configured to hold the optical scanning apparatus 15A at a predetermined position inside the distal end portion of the insertion section 11. Furthermore, for example, as shown in Figs. 9A and 9B, the holding member 17A has a concave groove section 171 provided on a surface, the groove section 171 having a width approximately the same as the length of the fixing member 16D in the Y-axis direction and formed along the X-axis direction. Note that it is enough if the groove section 171 is formed along one of the X-axis direction and the Y-axis direction.

The actuator section 18 includes at least one piezoelectric element configured to swing, in the X-axis direction, the exit end portion of the illumination fiber 12 protruding from the distal end portion of the fixing member 16D, and at least one piezoelectric element configured to swing, in the Y-axis direction, the exit end portion of the illumination fiber 12 protruding from the distal end portion of the fixing member 16D. More specifically, as shown in Figs. 9A and 9B, for example, the actuator section 18 includes the piezoelectric elements 181 and 182 provided in the X-axis direction, and the piezoelectric elements 183 and 184 provided in the Y-axis direction.

The piezoelectric elements 181 and 182 are provided at positions, on outer side surfaces of the fixing member 16D, facing each other across the illumination fiber 12. Also, the piezoelectric elements 181 and 182 are capable of swinging, in the X-axis direction, the exit end portion of the illumination fiber 12 protruding from the distal end portion of the fixing member 16D, by vibrating (repeatedly expanding and contracting while maintaining opposite expansion/contraction states) according to a first drive signal supplied by the driver unit 22 of the main body apparatus 3.

The piezoelectric elements 183 and 184 are provided at positions, on outer side surfaces of the fixing member 16D, facing each other across the illumination fiber 12. Also, the piezoelectric elements 183 and 184 are capable of swinging, in the Y-axis direction, the exit end portion of the illumination fiber 12 protruding from the distal end portion of the fixing member 16D, by vibrating (repeatedly expanding and contracting while maintaining opposite expansion/contraction states) according to a second drive signal supplied by the driver unit 22 of the main body apparatus 3.

The piezoelectric elements 181 to 184 are formed as cuboids having same length L, width W, and thickness T as one another. Also, the piezoelectric elements 181 to 184 are formed using a same piezoelectric material as one another.

The holding member 17A of the present embodiment is formed in such a way that the shape seen in the X-axis direction from the center axis passing though the center of the through hole 161 and the shape seen in the Y-axis direction from the center axis are asymmetric. Therefore, according to the present embodiment, the position of the fixing end for vibration of the piezoelectric elements 181 and 182 and the position of the fixing end for vibration of the piezoelectric element 183 and 184 may be made substantially different. That is, according to the present embodiment, the frequency f1 of the first drive signal may be made to coincide with the mechanical resonance frequency frx, in the X-axis direction, of the vibration of the piezoelectric elements 181 and 182, and also, the frequency f2 of the second drive signal may be made to coincide with the mechanical resonance frequency fry, in the Y-axis direction, of the vibration of the piezoelectric elements 183 and 184.

Therefore, according to the present embodiment, the sizes of the amplitudes of the drive signals at the time of swinging the illumination fiber 12 at desired amplitudes and in the Lissajous pattern may be optimized, and as a result, the efficiency at the time of scanning an object in the Lissajous scan pattern may be increased.

### (Third Embodiment)

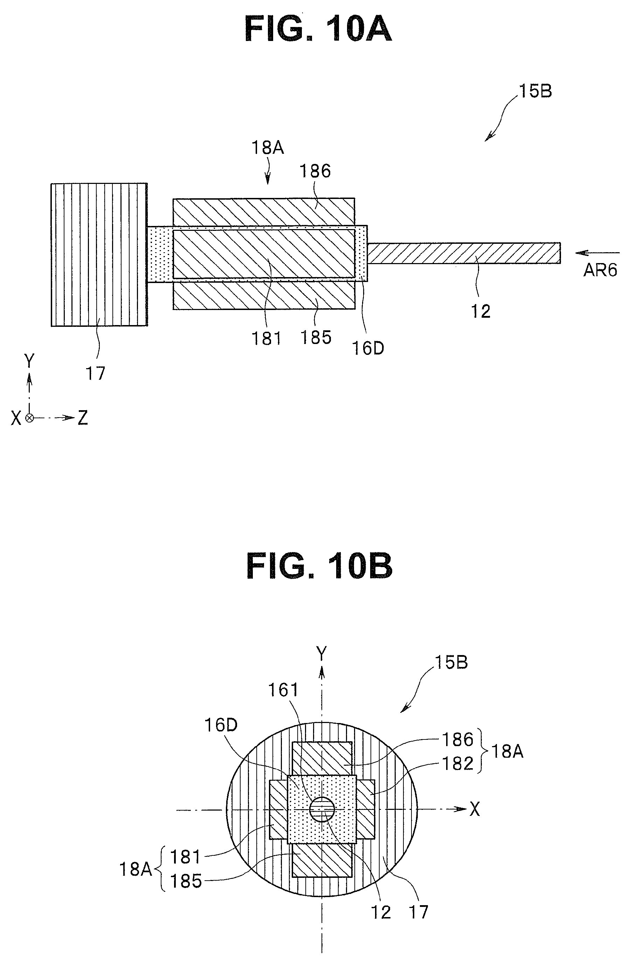

Figs. 10A to 15B are related to a third embodiment of the present invention.

Note that, in the present embodiment, sections having a configuration different from those of the first and the second embodiments will be mainly described while omitting as appropriate detailed description regarding sections having the same configuration as in at least the first embodiment or the second embodiment.

The scanning endoscope 2 of the present embodiment is configured by including an optical scanning apparatus 15B as shown in Figs. 10A and 10B at the distal end portion of the insertion section 11, instead of the optical scanning apparatus 15 and the optical scanning apparatus 15A.

More specifically, as shown in Figs. 10A and 10B, the optical scanning apparatus 15B includes the fixing member 16D formed as a square column, the holding member 17 configured to hold the fixing member 16D in a cantilevered manner at a proximal end portion of the fixing member 16D, and an actuator section 18A provided on outer side surfaces of the fixing member 16D. Fig. 10A is a diagram showing an example configuration of the optical scanning apparatus according to the third embodiment. Fig. 10B is a diagram showing an example configuration seen from a direction indicated by an arrow AR6 in Fig. 10A.

The holding member 17 functions as a fixing end of the optical scanning apparatus 15B, and is configured to hold the optical scanning apparatus 15B at a predetermined position inside the distal end portion of the insertion section 11.

The actuator section 18A includes at least one piezoelectric element configured to swing, in the X-axis direction, the exit end portion of the illumination fiber 12 protruding from the distal end portion of the fixing member 16D, and at least one piezoelectric element configured to swing, in the Y-axis direction, the exit end portion of the illumination fiber 12 protruding from the distal end portion of the fixing member 16D. More specifically, as shown in Figs. 10A and 10B, for example, the actuator section 18A includes the piezoelectric elements 181 and 182 provided in the X-axis direction, and piezoelectric elements 185 and 186 provided in the Y-axis direction.

The piezoelectric elements 181 and 182 are provided at positions, on outer side surfaces of the fixing member 16D, facing each other across the illumination fiber 12. Also, the piezoelectric elements 181 and 182 are capable of swinging, in the X-axis direction, the exit end portion of the illumination fiber 12 protruding from the distal end portion of the fixing member 16D, by vibrating (repeatedly expanding and contracting while maintaining opposite expansion/contraction states) according to a first drive signal supplied by the driver unit 22 of the main body apparatus 3. Moreover, the piezoelectric elements 181 and 182 are formed as cuboids having same length L1, width W1, and thickness T1 as each other.

The piezoelectric elements 185 and 186 are provided at positions, on outer side surfaces of the fixing member 16D, facing each other across the illumination fiber 12. Also, the piezoelectric elements 185 and 186 are capable of swinging, in the Y-axis direction, the exit end portion of the illumination fiber 12 protruding from the distal end portion of the fixing member 16D, by vibrating (repeatedly expanding and contracting while maintaining opposite expansion/contraction states) according to a second drive signal supplied by the driver unit 22 of the main body apparatus 3. Moreover, the piezoelectric elements 185 and 186 are formed as cuboids having same length L1, width W1, and thickness T2 as each other.

The piezoelectric elements 181, 182, 185, and 186 are formed using a same piezoelectric material as one another.

Furthermore, the actuator section 18A is formed in such a way that the thickness T1 of the piezoelectric elements 181 and 182 in the X-axis direction and the thickness T2 of the piezoelectric elements 185 and 186 in the Y-axis direction are different. That is, the actuator section 18A of the present embodiment is formed in such a way that the shape seen in the X-axis direction from the center axis passing though the center of the through hole 161 and the shape seen in the Y-axis direction from the center axis are asymmetric. Therefore, according to the present embodiment, the frequency f1 of the first drive signal may be made to coincide with the mechanical resonance frequency frx, in the X-axis direction, of the vibration of the piezoelectric elements 181 and 182, and also, the frequency f2 of the second drive signal may be made to coincide with mechanical resonance frequency frya, in the Y-axis direction, of the vibration of the piezoelectric elements 185 and 186.

Therefore, according to the present embodiment, the sizes of the amplitudes of the drive signals at the time of swinging the illumination fiber 12 at desired amplitudes and in the Lissajous pattern may be optimized, and as a result, the efficiency at the time of scanning an object in the Lissajous scan pattern may be increased.

Next, a configuration according to an example modification of the optical scanning apparatus 15B of the present embodiment will be described. Note that, in the following, for the sake of simplicity, sections different from those of the configuration described above will be mainly described while omitting as appropriate description regarding sections to which the same configuration as the configuration described above can be applied.

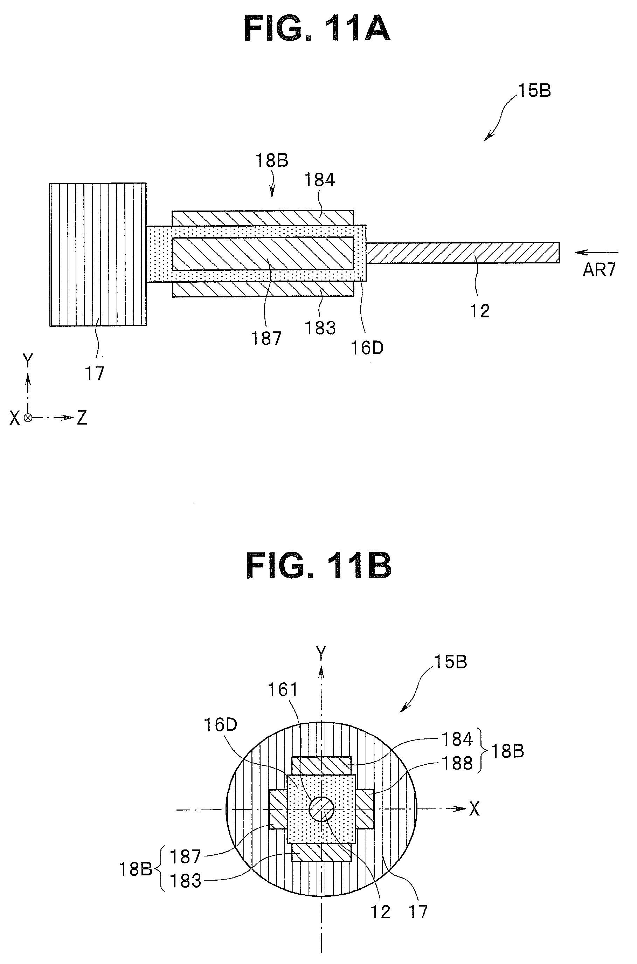

According to a first example modification of the present embodiment, the optical scanning apparatus 15B may be configured by including an actuator section 18B having a configuration as shown in Figs. 11A and 11B, instead of the actuator section 18A. Fig. 11A is a diagram showing an example configuration of the optical scanning apparatus according to the example modification of the third embodiment. Fig. 11B is a diagram showing an example configuration seen from a direction indicated by an arrow AR7 in Fig. 11 A.

The actuator section 18B includes at least one piezoelectric element configured to swing, in the X-axis direction, the exit end portion of the illumination fiber 12 protruding from the distal end portion of the fixing member 16D, and at least one piezoelectric element configured to swing, in the Y-axis direction, the exit end portion of the illumination fiber 12 protruding from the distal end portion of the fixing member 16D. More specifically, as shown in Figs. 11A and 11B, for example, the actuator section 18B includes piezoelectric elements 187 and 188 provided in the X-axis direction, and the piezoelectric elements 183 and 184 provided in the Y-axis direction.

The piezoelectric elements 187 and 188 are provided at positions, on outer side surfaces of the fixing member 16D, facing each other across the illumination fiber 12. Also, the piezoelectric elements 187 and 188 are capable of swinging, in the X-axis direction, the exit end portion of the illumination fiber 12 protruding from the distal end portion of the fixing member 16D, by vibrating (repeatedly expanding and contracting while maintaining opposite expansion/contraction states) according to a first drive signal supplied by the driver unit 22 of the main body apparatus 3. Moreover, the piezoelectric elements 187 and 188 are formed as cuboids having same length L1, width W2, and thickness T1 as each other.

The piezoelectric elements 183 and 184 are provided at positions, on outer side surfaces of the fixing member 16D, facing each other across the illumination fiber 12. Also, the piezoelectric elements 183 and 184 are capable of swinging, in the Y-axis direction, the exit end portion of the illumination fiber 12 protruding from the distal end portion of the fixing member 16D, by vibrating (repeatedly expanding and contracting while maintaining opposite expansion/contraction states) according to a second drive signal supplied by the driver unit 22 of the main body apparatus 3. Moreover, the piezoelectric elements 183 and 184 are formed as cuboids having same length L1, width W1, and thickness T1 as each other.

The piezoelectric elements 183, 184, 187, and 188 are formed using a same piezoelectric material as one another.

Furthermore, the actuator section 18B is formed such that the width W1 of the piezoelectric elements 183 and 184 in the X-axis direction and the width W2 of the piezoelectric elements 187 and 188 in the Y-axis direction are different. That is, the actuator section 18B of the present embodiment is formed in such a way that the shape seen in the X-axis direction from the center axis passing though the center of the through hole 161 and the shape seen in the Y-axis direction from the center axis are asymmetric. Therefore, according to the present example modification, the frequency f1 of the first drive signal may be made to coincide with mechanical resonance frequency frxb, in the X-axis direction, of the vibration of the piezoelectric elements 187 and 188, and also, the frequency f2 of the second drive signal may be made to coincide with the mechanical resonance frequency fry, in the Y-axis direction, of the vibration of the piezoelectric elements 183 and 184.

Therefore, according to the present example modification, the sizes of the amplitudes of the drive signals at the time of swinging the illumination fiber 12 at desired amplitudes and in the Lissajous pattern may be optimized, and as a result, the efficiency at the time of scanning an object in the Lissajous scan pattern may be increased.

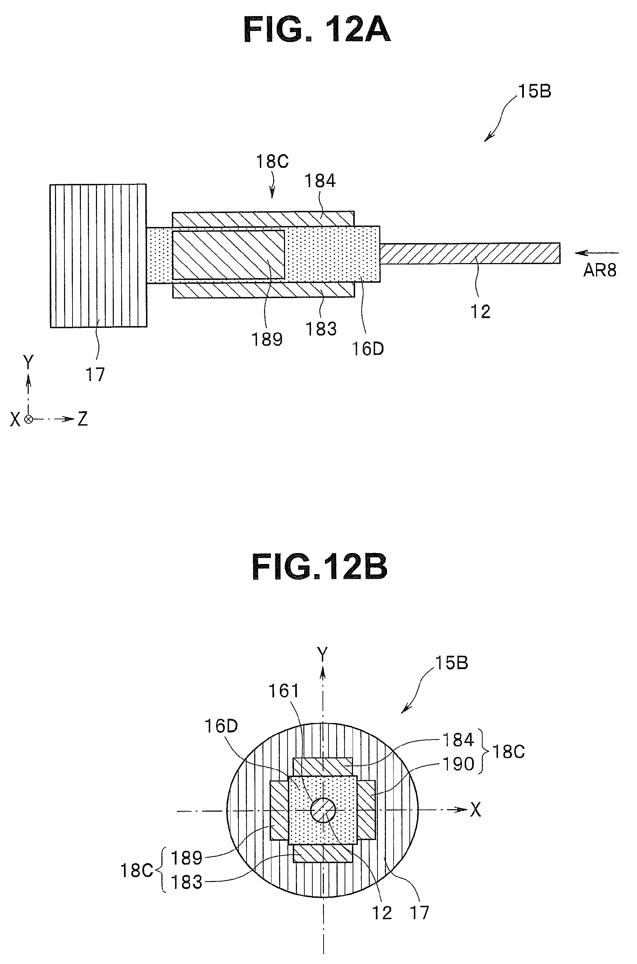

According to a second example modification of the present embodiment, the optical scanning apparatus 15B may be configured by including an actuator section 18C having a configuration as shown in Figs. 12A and 12B, instead of the actuator section 18A. Fig. 12A is a diagram showing an example configuration of the optical scanning apparatus according to the example modification of the third embodiment, different from that shown in Fig. 11A. Fig. 12B is a diagram showing an example configuration seen from a direction indicated by an arrow AR8 in Fig. 12A.

The actuator section 18C includes at least one piezoelectric element configured to swing, in the X-axis direction, the exit end portion of the illumination fiber 12 protruding from the distal end portion of the fixing member 16D, and at least one piezoelectric element configured to swing, in the Y-axis direction, the exit end portion of the illumination fiber 12 protruding from the distal end portion of the fixing member 16D. More specifically, as shown in Figs. 12A and 12B, for example, the actuator section 18C includes piezoelectric elements 189 and 190 provided in the X-axis direction, and the piezoelectric elements 183 and 184 provided in the Y-axis direction.

The piezoelectric elements 189 and 190 are provided at positions, on outer side surfaces of the fixing member 16D, facing each other across the illumination fiber 12. Also, the piezoelectric elements 189 and 190 are capable of swinging, in the X-axis direction, the exit end portion of the illumination fiber 12 protruding from the distal end portion of the fixing member 16D, by vibrating (repeatedly expanding and contracting while maintaining opposite expansion/contraction states) according to a first drive signal supplied by the driver unit 22 of the main body apparatus 3. Furthermore, the piezoelectric elements 189 and 190 are formed as cuboids having same length L2, width W1, and thickness T1 as each other.

The piezoelectric elements 183 and 184 are provided at positions, on outer side surfaces of the fixing member 16D, facing each other across the illumination fiber 12. Also, the piezoelectric elements 183 and 184 are capable of swinging, in the Y-axis direction, the exit end portion of the illumination fiber 12 protruding from the distal end portion of the fixing member 16D, by vibrating (repeatedly expanding and contracting while maintaining opposite expansion/contraction states) according to a second drive signal supplied by the driver unit 22 of the main body apparatus 3. Furthermore, the piezoelectric elements 185 and 186 are formed as cuboids having same length L1, width W1, and thickness T1 as each other.

The piezoelectric elements 183, 184, 189, and 190 are formed using a same piezoelectric material as one another.

Furthermore, the actuator section 18C is formed such that the length L1 of the piezoelectric elements 183 and 184 in the Z-axis direction and the length L2 of the piezoelectric elements 189 and 190 in the Z-axis direction are different. That is, the actuator section 18C of the present embodiment is formed in such a way that the shape seen in the X-axis direction from the center axis passing though the center of the through hole 161 and the shape seen in the Y-axis direction from the center axis are asymmetric. Therefore, according to the present example modification, the frequency f1 of the first drive signal may be made to coincide with mechanical resonance frequency frxc, in the X-axis direction, of the vibration of the piezoelectric elements 189 and 190, and also, the frequency f2 of the second drive signal may be made to coincide with the mechanical resonance frequency fry, in the Y-axis direction, of the vibration of the piezoelectric elements 183 and 184.

Therefore, according to the present example modification, the sizes of the amplitudes of the drive signals at the time of swinging the illumination fiber 12 at desired amplitudes and in the Lissajous pattern may be optimized, and as a result, the efficiency at the time of scanning an object in the Lissajous scan pattern may be increased.

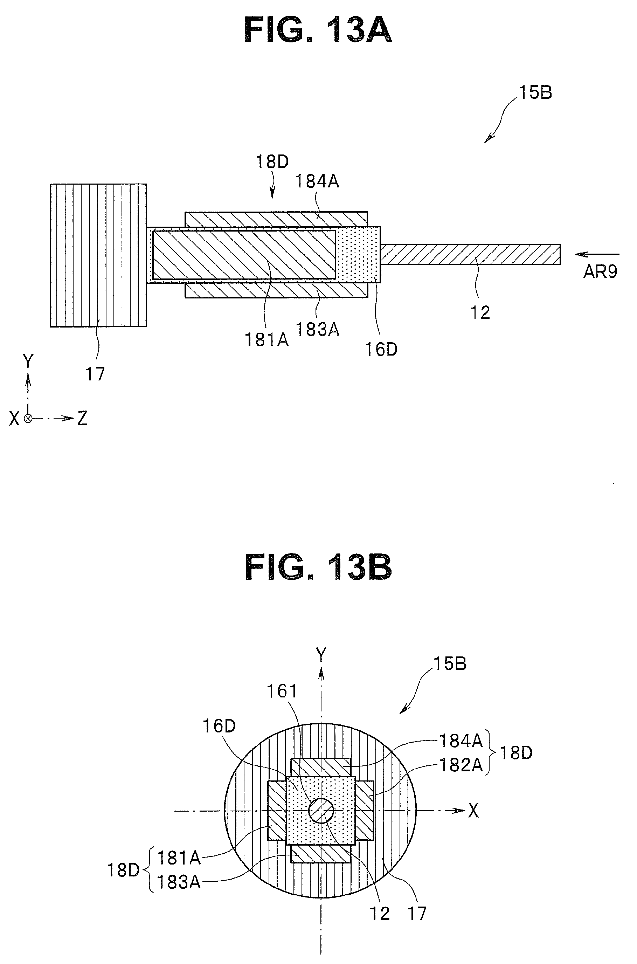

According to a third example modification of the present embodiment, the optical scanning apparatus 15B may be configured by including an actuator section 18D having a configuration as shown in Figs. 13A and 13B, instead of the actuator section 18A. Fig. 13A is a diagram showing an example configuration of the optical scanning apparatus according to the example modification of the third embodiment, different from those shown in Figs. 11A and 12A. Fig. 13B is a diagram showing an example configuration seen from a direction indicated by an arrow AR9 in Fig. 13A.

The actuator section 18D includes at least one piezoelectric element configured to swing, in the X-axis direction, the exit end portion of the illumination fiber 12 protruding from the distal end portion of the fixing member 16D, and at least one piezoelectric element configured to swing, in the Y-axis direction, the exit end portion of the illumination fiber 12 protruding from the distal end portion of the fixing member 16D. More specifically, as shown in Figs. 13A and 13B, for example, the actuator section 18D includes piezoelectric elements 181A and 182A provided in the X-axis direction, and piezoelectric elements 183A and 184A provided in the Y-axis direction.

The piezoelectric elements 181A and 182A are provided at positions, on outer side surfaces on a proximal end portion side of the fixing member 16D, facing each other across the illumination fiber 12. Also, the piezoelectric elements 181A and 182A are capable of swinging, in the X-axis direction, the exit end portion of the illumination fiber 12 protruding from the distal end portion of the fixing member 16D, by vibrating (repeatedly expanding and contracting while maintaining opposite expansion/contraction states) according to a first drive signal supplied by the driver unit 22 of the main body apparatus 3.

The piezoelectric elements 183A and 184A are provided at positions, on outer side surfaces on a distal end portion side of the fixing member 16D, facing each other across the illumination fiber 12. Also, the piezoelectric elements 183 and 184 are capable of swinging, in the Y-axis direction, the exit end portion of the illumination fiber 12 protruding from the distal end portion of the fixing member 16D, by vibrating (repeatedly expanding and contracting while maintaining opposite expansion/contraction states) according to a second drive signal supplied by the driver unit 22 of the main body apparatus 3.

The piezoelectric elements 181A to 184A are formed as cuboids having same length L, width W, and thickness T as one another. Also, the piezoelectric elements 181A to 184A are formed using a same piezoelectric material as one another.

As described above, the actuator section 18D is configured with arranged positions of the piezoelectric elements 181A and 182A on the outer side surfaces of the fixing member 16D and arranged positions of the piezoelectric elements 183A and 184A on the outer side surfaces of the fixing member 16D shifted from each other along the Z-axis direction. That is, the actuator section 18D of the present embodiment is formed in such a way that the shape seen in the X-axis direction from the center axis passing though the center of the through hole 161 and the shape seen in the Y-axis direction from the center axis are asymmetric.

Therefore, according to the present example modification, the frequency f1 of the first drive signal may be made to coincide with mechanical resonance frequency frxd, in the X-axis direction, of the vibration of the piezoelectric elements 181A and 182A, and also, the frequency f2 of the second drive signal may be made to coincide with mechanical resonance frequency fryd, in the Y-axis direction, of the vibration of the piezoelectric elements 183A and 184A.

Therefore, according to the present example modification, the sizes of the amplitudes of the drive signals at the time of swinging the illumination fiber 12 at desired amplitudes and in the Lissajous pattern may be optimized, and as a result, the efficiency at the time of scanning an object in the Lissajous scan pattern may be increased.

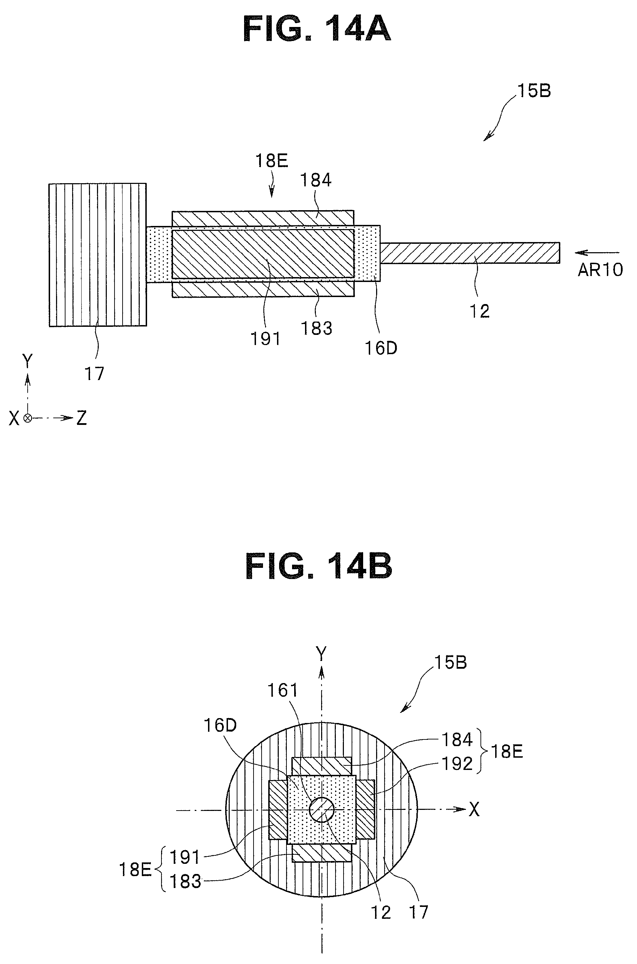

According to a fourth example modification of the present embodiment, the optical scanning apparatus 15B may be configured by including an actuator section 18E having a configuration as shown in Figs. 14A and 14B, instead of the actuator section 18A. Fig. 14A is a diagram showing an example configuration of the optical scanning apparatus according to the example modification of the third embodiment, different from those shown in Figs. 11A, 12A, and 13A. Fig. 14B is a diagram showing an example configuration seen from a direction indicated by an arrow AR10 in Fig. 14A.

The actuator section 18E includes at least one piezoelectric element configured to swing, in the X-axis direction, the exit end portion of the illumination fiber 12 protruding from the distal end portion of the fixing member 16D, and at least one piezoelectric element configured to swing, in the Y-axis direction, the exit end portion of the illumination fiber 12 protruding from the distal end portion of the fixing member 16D. More specifically, as shown in Figs. 14A and 14B, for example, the actuator section 18E includes piezoelectric elements 191 and 192 provided in the X-axis direction, and the piezoelectric elements 183 and 184 provided in the Y-axis direction.

The piezoelectric elements 191 and 192 are provided at positions, on outer side surfaces of the fixing member 16D, facing each other across the illumination fiber 12. Also, the piezoelectric elements 191 and 192 are capable of swinging, in the X-axis direction, the exit end portion of the illumination fiber 12 protruding from the distal end portion of the fixing member 16D, by vibrating (repeatedly expanding and contracting while maintaining opposite expansion/contraction states) according to a first drive signal supplied by the driver unit 22 of the main body apparatus 3. Furthermore, the piezoelectric elements 191 and 192 are formed using a piezoelectric material that is the same between each other but different from that of the piezoelectric elements 183 and 184.

The piezoelectric elements 183 and 184 are provided at positions, on outer side surfaces of the fixing member 16D, facing each other across the illumination fiber 12. Also, the piezoelectric elements 183 and 184 are capable of swinging, in the Y-axis direction, the exit end portion of the illumination fiber 12 protruding from the distal end portion of the fixing member 16D, by vibrating (repeatedly expanding and contracting while maintaining opposite expansion/contraction states) according to a second drive signal supplied by the driver unit 22 of the main body apparatus 3. Furthermore, the piezoelectric elements 183 and 184 are formed using a piezoelectric material that is the same between each other but different from that of the piezoelectric elements 191 and 192.

The piezoelectric elements 183, 184, 191, and 192A are formed as cuboids having same length L, width W, and thickness T as one another.

As described above, according to the actuator section 18E, the piezoelectric material used for forming the piezoelectric elements 191 and 192 and the piezoelectric material used for forming the piezoelectric elements 183 and 184 are different from each other.

Therefore, according to the present example modification, the frequency f1 of the first drive signal may be made to coincide with mechanical resonance frequency frxe, in the X-axis direction, of the vibration of the piezoelectric elements 191 and 192, and also, the frequency f2, of the second drive signal may be made to coincide with the mechanical resonance frequency fry, in the Y-axis direction, of the vibration of the piezoelectric elements 183 and 184.

Therefore, according to the present example modification, the sizes of the amplitudes of the drive signals at the time of swinging the illumination fiber 12 at desired amplitudes and in the Lissajous pattern may be optimized, and as a result, the efficiency at the time of scanning an object in the Lissajous scan pattern may be increased.

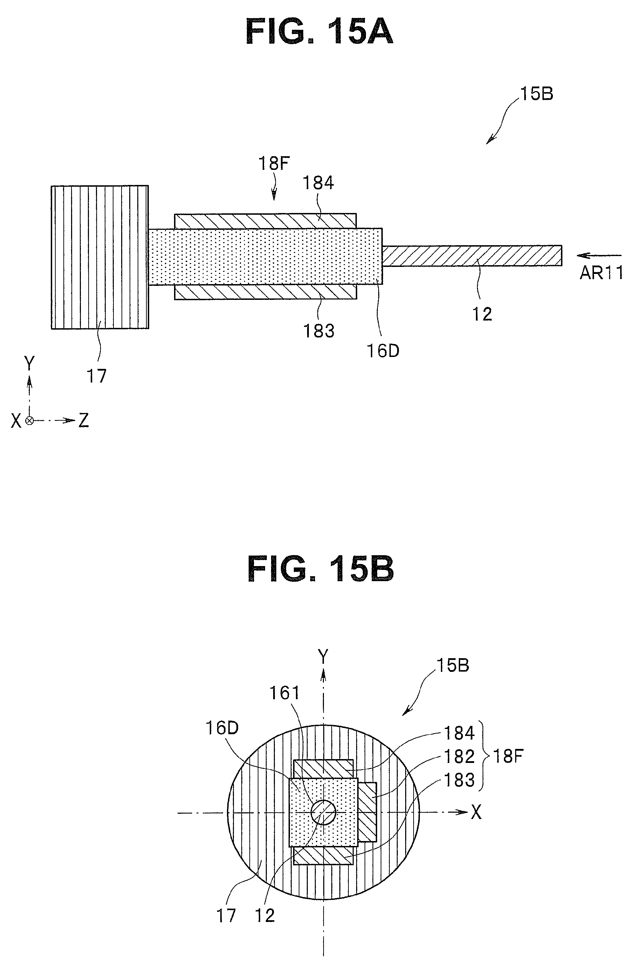

According to a fifth example modification of the present embodiment, the optical scanning apparatus 15B may be configured by including an actuator section 18F having a configuration as shown in Figs. 15A and 15B, instead of the actuator section 18A. Fig. 15A is a diagram showing an example configuration of the optical scanning apparatus according to the example modification of the third embodiment, different from those shown in Figs. 11 A, 12A, 13A, and 14A. Fig. 15B is a diagram showing an example configuration seen from a direction indicated by an arrow AR11 in Fig. 15A.

The actuator section 18F includes at least one piezoelectric element configured to swing, in the X-axis direction, the exit end portion of the illumination fiber 12 protruding from the distal end portion of the fixing member 16D, and at least one piezoelectric element configured to swing, in the Y-axis direction, the exit end portion of the illumination fiber 12 protruding from the distal end portion of the fixing member 16D. More specifically, as shown in Figs. 15A and 15B, for example, the actuator section 18F includes the piezoelectric element 182 provided in the X-axis direction, and the piezoelectric elements 183 and 184 provided in the Y-axis direction.

The piezoelectric element 182 is provided at positions, on outer side surfaces of the fixing member 16D, facing each other across the illumination fiber 12. Also, the piezoelectric element 182 is capable of swinging, in the X-axis direction, the exit end portion of the illumination fiber 12 protruding from the distal end portion of the fixing member 16D, by vibrating (repeatedly expanding and contracting while maintaining opposite expansion/contraction states) according to a first drive signal supplied by the driver unit 22 of the main body apparatus 3.

The piezoelectric elements 183 and 184 are provided at positions, on outer side surfaces of the fixing member 16D, facing each other across the illumination fiber 12. Also, the piezoelectric elements 183 and 184 are capable of swinging, in the Y-axis direction, the exit end portion of the illumination fiber 12 protruding from the distal end portion of the fixing member 16D, by vibrating (repeatedly expanding and contracting while maintaining opposite expansion/contraction states) according to a second drive signal supplied by the driver unit 22 of the main body apparatus 3.

The piezoelectric elements 182 to 184 are formed as cuboids having same length L, width W, and thickness T as one another. Furthermore, the piezoelectric elements 182 to 184 are formed using a same piezoelectric material as one another.

As described above, according to the actuator section 18F, the number of piezoelectric elements provided in the X-axis direction and the number of piezoelectric elements provided in the Y-axis direction are different from each other. That is, the actuator section 18F of the present embodiment is formed in such a way that the shape seen in the X-axis direction from the center axis passing though the center of the through hole 161 and the shape seen in the Y-axis direction from the center axis are asymmetric.

Therefore, according to the present example modification, the frequency f1 of the first drive signal may be made to coincide with mechanical resonance frequency frxf, in the X-axis direction, of the vibration of the piezoelectric element 182, and also, the frequency f2 of the second drive signal may be made to coincide with the mechanical resonance frequency fry, in the Y-axis direction, of the vibration of the piezoelectric elements 183 and 184.

Therefore, according to the present example modification, the sizes of the amplitudes of the drive signals at the time of swinging the illumination fiber 12 at desired amplitudes and in the Lissajous pattern may be optimized, and as a result, the efficiency at the time of scanning an object in the Lissajous scan pattern may be increased.

Note that the present invention is not limited to each of the embodiments and example modifications described above, and may be subjected to various changes and alterations within the scope of the invention.

The present application is filed claiming priority based on Japanese Patent Application No. 2014-088464 filed in Japan on April 22, 2014, and the above described disclosure is incorporated by reference in the present description, claims and drawings.

## Claims

1. An optical scanning apparatus comprising:
a fixing member having, at a center portion, a cylindrical through hole allowing penetration of a light guide member that is configured to guide light entering an incident end portion and to emit the light from an exit end portion, the fixing member being configured to fix the exit end portion in a state penetrating through the through hole;
a holding member configured to hold the fixing member at a proximal end portion of the fixing member in a cantilevered manner; and
an actuator section including a first drive section, provided on an outer surface of the fixing member, configured to swing, in a first direction, the exit end portion protruding from a distal end portion of the fixing member, and a second drive section, provided on an outer surface of the fixing member, configured to swing, in a second direction different from the first direction, the exit end portion protruding from the distal end portion of the fixing member,
wherein at least one of the fixing member, the holding member, and the actuator section includes a part that is formed in such a way that a shape seen in the first direction from a center axis passing through a center of the through hole and a shape seen in the second direction from the center axis are asymmetric.

2. The optical scanning apparatus according to claim 1, wherein the fixing member is configured to have a shape whose length in the first direction and length in the second direction are different from each other.

3. The optical scanning apparatus according to claim 1, wherein the through hole is formed to have a shape whose length in the first direction and length in the second direction are different from each other.

4. The optical scanning apparatus according to claim 1, wherein
the first drive section is configured by including a piezoelectric element that is formed to have a predetermined shape, and
the second drive section is configured by including a piezoelectric element that is formed to have a shape different from the predetermined shape.

5. The optical scanning apparatus according to claim 1, wherein
the first drive section is configured by including a predetermined number of piezoelectric elements, and
the second drive section is configured by including a number, different from the predetermined number, of piezoelectric elements.

6. The optical scanning apparatus according to claim 1, wherein
the first drive section is configured by including two piezoelectric elements that are provided at positions facing each other across the light guide member,
the second drive section is configured by including two piezoelectric elements that are provided at positions facing each other across the light guide member, and
the actuator section is configured with arranged positions of the two piezoelectric elements, of the first drive section, on outer surfaces of the fixing member and arranged positions of the two piezoelectric elements, of the second drive section, on outer surfaces of the fixing member shifted from each other along a direction of the center axis.

7. The optical scanning apparatus according to claim 1, wherein the second direction is a direction orthogonal to the first direction.

8. The optical scanning apparatus according to claim 7, wherein the center axis is orthogonal to each of the first direction and the second direction.

9. A scanning endoscope comprising:
an insertion section formed to have a shape allowing insertion into a body cavity;
a light guide member, inserted through the insertion section, configured to guide light entering an incident end portion and to emit the light from an exit end portion;
a fixing member having, at a center portion, a cylindrical through hole allowing penetration of the light guide member, the fixing member being configured to fix the exit end portion in a state penetrating through the through hole;
a holding member configured to hold the fixing member at a proximal end portion of the fixing member in a cantilevered manner; and
an actuator section including a first drive section, provided on an outer surface of the fixing member, configured to swing, in a first direction, the exit end portion protruding from a distal end portion of the fixing member, and a second drive section, provided on an outer surface of the fixing member, configured to swing, in a second direction different from the first direction, the exit end portion protruding from the distal end portion of the fixing member,
wherein at least one of the fixing member, the holding member, and the actuator section includes a part that is formed in such a way that a shape seen in the first direction from a center axis passing through a center of the through hole and a shape seen in the second direction from the center axis are asymmetric.
